# EUROPÄISCHE PATENTSCHRIFT

(11) **EP 2 204 148 B1**
(45) Veröffentlichungstag und Bekanntmachung des Hinweises auf die Patenterteilung: **28.03.2012**
(21) Anmeldenummer: 08106040.2
(22) Anmeldetag: 30.12.2008
(51) Int. Cl.: A61F 5/56

(54) **Implantat zur Behandlung des obstruktiven Schlafapnoesyndroms**
Implant for treatment of obstructive sleep apnea
Implant de traitement d'une apnée du sommeil obstruante

(43) Veröffentlichungstag der Anmeldung: 07.07.2010
(73) Patentinhaber: Medartis AG, 4057 Basel (CH)
(72) Erfinder: Tschopp, Kurt, 4410 Liestal (CH); Breitenstein, Michael, 4493 Wenslingen (CH)
(74) Vertreter: Hepp Wenger Ryffel AG

(56) Entgegenhaltungen:
- EP-B1- 1 216 013
- WO-A-2007/095582

## Beschreibung

Die vorliegende Erfindung betrifft ein Implantat für einen insbesondere menschlichen Gaumen, ein Implantatset sowie ein Auswahlverfahren zum Auswählen eines Implantats gemäss den Oberbegriffen der unabhängigen Patentansprüche.

Etwa 2-4% der Weltbevölkerung im mittleren Lebensalter leiden an einem obstruktiven Schlafapnoesyndrom (OSAS). Die hiervon betroffenen Patienten leiden an Erschlaffungen im Rachen- und Halsraum, welche zu meist kurzen Atemstillständen führen, die sich unter Umständen mehrere hundert Male pro Nacht wiederholen. Die obstruktive Schlafapnoe ist ein Risikofaktor für Herz-Kreislauferkrankungen wie Herzinfarkte oder Hirnschläge. Die gehäuften Atemstillstände können im schlimmsten Falle zu einem plötzlichen Tod durch Herzstillstand oder Hirnschlag führen. Als weitere Folge der Atemaussetzer sind Aufweckreaktionen zu nennen, die zu einem gestörten, zerhackten Schlaf ohne Tiefschlafphasen führen. Die Folge davon ist, dass die Patienten am Morgen nicht ausgeruht sind und über eine verstärkte Tagesmüdigkeit klagen. Besonders gefährlich ist der so genannte Sekundenschlaf, der zum Beispiel beim Autofahren auftreten kann. So ist das Risiko für einen tödlichen Autounfall bei Patienten mit OSAS siebenfach erhöht gegenüber der Normalbevölkerung.

Auch das Schnarchen beruht auf ähnlichen anatomischen Gegebenheiten, nämlich einer Verengung der Atemwege im Schlaf. Allerdings ist der Kollaps der Atemwege nicht vollständig, sondern nur teilweise, so dass lediglich die Entwicklung von Schnarchgeräuschen stattfindet, nicht jedoch ein Sauerstoffabfall im Blut oder eine Aufweckreaktion. Schnarchen bedroht zwar deshalb den Patienten selbst nicht, kann jedoch das persönliche Umfeld in Mitleidenschaft ziehen.

Der Kollaps der Atemwege kann entweder durch einen Verschluss auf Höhe des Gaumens oder durch ein Zurückfallen der Zunge bedingt sein. Um das Krankheitsbild zu behandeln, werden seit einiger Zeit Implantate verwendet, welche entweder in den weichen Teil des Gaumens oder den Zungengrund eingesetzt werden. Ein derartiges, in den weichen Gaumen einsetzbares Implantat ist beispielsweise in EP 1 216 013 beschrieben. Dieses Implantat besteht etwa aus miteinander verflochtenen Polyester-Fasern. Wird eines oder mehrere dieser Implantate in den weichen Gaumen des Patienten eingesetzt, so führt dies zu einer Versteifung des weichen Gaumens. Hierdurch soll das dynamische Verhalten des weichen Gaumens derart verändert werden, dass eine wie beim Schnarchen auftretende Flatterbewegung sowie das Zurückfallen des weichen Gaumens in einer liegenden Position des Patienten verhindert werden können.

Dieses aus dem Stand der Technik bekannte Implantat weist jedoch eine Reihe von Nachteilen auf. Denn selbst wenn der gesamte weiche Gaumen versteift wird, so kann er dennoch als Ganzes eine Flatterbewegung durchführen und auch als Ganzes in einer liegenden Position des Patienten in den Rachenraum zurückfallen, was zu gefährlichen Atemstillständen führen kann.

Ein weiteres Implantat gemäß des Oberbegriffes von Anspruch 1 ist aus der Patentschrift WO2007/095582 bekannt.

Es ist eine Aufgabe der vorliegenden Erfindung, die Nachteile aus dem Stand der Technik zu überwinden und insbesondere ein Implantat für einen Gaumen bereitzustellen, welches die zum Schnarchen führenden Flatterbewegungen des Gaumens wirkungsvoll unterdrückt und auch ein Zurückfallen des weichen Gaumens verhindert, ohne dass beispielsweise das Sprechen oder Schlucken behindert werden.

Diese Aufgabe wird gelöst durch ein Implantat, ein Implantatset und ein Verfahren mit den Merkmalen der kennzeichnenden Teile der unabhängigen Patentansprüche. Das Implantat ist geeignet für einen insbesondere menschlichen Gaumen. Es enthält einen distalen Abschnitt, welcher derart dimensioniert ist, dass er vollständig in einem weichen Gaumen aufnehmbar ist. Der Begriff "distal" bezieht sich auf die Position des Abschnitts bei bestimmungsgemässer Einsetzung des Implantats in einen Gaumen.

Erfindungsgemäss umfasst das Implantat weiterhin einen proximalen Abschnitt, welcher derart dimensioniert ist, dass er an einem harten Gaumen festlegbar ist. Auch der Begriff "proximal" bezieht sich auf die Position bei bestimmungsgemässer Einsetzung des Implantats in einen Gaumen. Zudem verfügt das Implantat über einen mittleren Abschnitt, der sich zwischen dem proximalen Abschnitt und dem distalen Abschnitt erstreckt.

Ein erfindungsgemässes Implantat kann in einen Gaumen eingesetzt werden, indem der proximale Abschnitt an einem harten Gaumen festgelegt wird, insbesondere entlang der gesamten Länge des proximalen Abschnitts. Der distale Abschnitt kann vollständig in einen weichen Gaumen eingesetzt werden. Aufgrund der Festlegung am harten Gaumen kann zumindest der proximale Abschnitt, insbesondere das gesamte Implantat, im Wesentlichen winkelstabil relativ zum harten Gaumen befestigt werden. Der distale Abschnitt dient der Versteifung des weichen Gaumens und schränkt eine Flatterbewegung oder eine sonstige Bewegung des weichen Gaumens ein.

Aufgrund der Festlegung des proximalen Abschnitts am harten Gaumen wird zusätzlich auch die Bewegung des weichen Gaumens als Ganzes relativ zum harten Gaumen eingeschränkt. Das erfindungsgemässe Implantat dient somit effektiv der Verlängerung des harten Gaumens und der Einnahme einer natürlichen Ruheposition, sofern der weiche Gaumen nicht durch Muskelanspannung der Gaumenmuskulatur in eine andere Position gebracht wird. Somit wird gegenüber dem Stand der Technik das Risiko eines Zurückfallens des weichen Gaumens in einer liegenden Position eines Patienten deutlich verringert.

Das Implantat kann entweder bereits in einer Einsetzform vorliegen, oder es kann zumindest in eine Einsetzform gebracht werden. Unter der Einsetzform wird dabei die Form des Implantats verstanden, wenn es bestimmungsgemäss in einen Gaumen eingesetzt ist und dabei der proximale Abschnitt am harten Gaumen festgelegt und der distale Abschnitt vollständig im weichen Gaumen aufgenommen ist. Zumindest in dieser Einsetzform kann der distale Abschnitt in einem Winkel relativ zum proximalen Abschnitt angeordnet sein.

In einer sich durch den proximalen Abschnitt und den distalen Abschnitt erstreckenden Schnittebene kann der mittlere Abschnitt in jedem Punkt eine Krümmung aufweisen. Unter einer Krümmung an einem Punkt des Implantats wird hier und im Folgenden der Kehrwert des Radius eines imaginären Schmiegekreises verstanden, welcher sich in diesem Punkt an den mittleren Abschnitt anschmiegt. Eine derartige Krümmung hat also die Dimension einer inversen Länge.

Bevorzugt ist zumindest in der Einsetzform des Implantats die Krümmung des mittleren Abschnitts in der Schnittebene in jedem seiner Punkte grösser als die Krümmung des proximalen Abschnitts in jedem von dessen Punkten. Weiterhin bevorzugt ist zumindest in der Einsetzform des Implantats die Krümmung des mittleren Abschnitts in jedem seiner Punkte grösser als die Krümmung des distalen Abschnitts in jedem von dessen Punkten. Die Krümmung des proximalen Abschnitts und des distalen Abschnitts sind dabei so definiert wie die Krümmung des mittleren Abschnitts.

Mit anderen Worten enthält das Implantat also bevorzugt zwei Abschnitte (einen proximalen Abschnitt und einen distalen Abschnitt), die zumindest in der Einsetzform des Implantats eine kleinere Krümmung aufweisen als ein sich dazwischen erstreckender mittlerer Abschnitt.

Insbesondere ist es möglich und liegt im Rahmen der obigen Definition, dass zumindest in der Einsetzform der proximale Abschnitt und/oder der distale Abschnitt gerade, d.h. ungekrümmt sind; in diesem Falle wird jedem Punkt des proximalen Abschnitts und/oder des distalen Abschnitts eine Krümmung von null zugewiesen. Weiterhin liegt es im Rahmen der obigen Definition, dass zumindest in der Einsetzform der mittlere Abschnitt mindestens einen Knick umfasst oder aus mindestens einem Knick besteht. Einem Knick wird dabei formal eine Krümmung von unendlich zugewiesen.

Die Abstimmung der Krümmungen des proximalen, des distalen und des mittleren Abschnitts in der Einsetzform bewirken, dass der proximale Abschnitt und/oder der distale Abschnitt im Wesentlichen ungekrümmt oder nur schwach gekrümmt verlaufen können und folglich der Anatomie des harten Gaumens bzw. des weichen Gaumens entsprechen. Der mittlere Abschnitt des Implantats kann in der Nähe des Übergangs vom harten Gaumen zum weichen Gaumen angeordnet sein, wo die Anatomie in natürlicher Weise eine grössere Krümmung vorgibt.

Bevorzugt beträgt in der Einsetzform die Krümmung des proximalen Abschnitts in der Schnittebene in jedem Punkt höchstens 1 cm⁻¹, bevorzugt 0,5 cm⁻¹, besonders bevorzugt höchstens 0,2 cm⁻¹. Weiterhin bevorzugt beträgt in der Einsetzform die Krümmung des distalen Abschnitts in der Schnittebene in jedem Punkt höchstens 1 cm⁻¹, bevorzugt höchstens 0,5 cm⁻¹, besonders bevorzugt höchstens 0,2 cm⁻¹. Insbesondere können der proximale und/oder der distale Abschnitt in der Einsetzform gerade sein; gemäss obiger Definition liegt dann eine Krümmung von null vor.

Ebenfalls bevorzugt weist in der Einsetzform der mittlere Abschnitt in jedem Punkt eine Krümmung von mindestens 2 cm⁻¹, mindestens 10 cm⁻¹, besonders bevorzugt mindestens 100 cm⁻¹ auf. Insbesondere kann der mittlere Abschnitt in der Einsetzform an einem Punkt einen Knick aufweisen; im Rahmen der obigen Definition wird diesem Punkt dann eine Krümmung von unendlich zugewiesen.

In einigen Ausführungsformen ist das Implantat derart ausgebildet, dass es nicht nur in der Einsetzform vorliegen kann, sondern auch in einer Bereitstellungsform, von der aus es in die Einsetzform bringbar ist. In der Bereitstellungsform kann das Implantat qualitativ andere Krümmungseigenschaften aufweisen als in der Einsetzform. Beispielsweise kann es in der Bereitstellungsform flach, also vollständig ungekrümmt sein. In dieser Bereitstellungsposition kann das Implantat z. B. leichter verpackt und/oder leichter eingesetzt werden.

In einigen Ausführungsformen kann der mittlere Abschnitt einen Solldeformationsbereich aufweisen, an dem der mittlere Abschnitt insbesondere plastisch deformiert, insbesondere gebogen und/oder geknickt werden kann. Auf diese Weise wird eine bevorzugte Position des Implantats definiert, an welcher es gebogen und/oder geknickt werden kann. Das Implantat kann dann durch Deformation des Solldeformationsbereichs von der Bereitstellungsform, in welcher das Implantat beispielsweise flach sein könnte, in die Einsetzform gebracht werden. Bevorzugt umfasst der Solldeformationsbereich mindestens eine Einschnürung, an welcher der mittlere Abschnitt biegbar oder knickbar ist.

In einigen Ausführungsformen kann das Implantat ein Formgedächtnismaterial, insbesondere NITINOL, umfassen oder daraus bestehen. Ein solches Implantat kann beispielsweise derart ausgebildet sein, dass es bei einer Bereitstellungstemperatur in der Bereitstellungsform und bei einer Einsetztemperatur in der Einsetzform vorliegt. Demgemäss stimmt die Einsetztemperatur mit der üblichen menschlichen Körpertemperatur überein und liegt daher bevorzugt zwischen 35 °C und 40 °C, besonders bevorzugt zwischen 36 °C und 37 °C. Die Bereitstellungstemperatur kann höher oder niedriger als die Einsetztemperatur sein. Das Implantat kann bei einer Bereitstellungstemperatur in einer derartigen Bereitstellungsform vorliegen, in welcher es besonders einfach in einen Gaumen einsetzbar ist, beispielsweise in einer flachen Form. Aufgrund der Temperaturänderung, die das Implantat beim Einsetzen erfährt, nimmt es dann von selbst die Einsetzform an. Die Herstellung von Implantaten an sich, die ein Formgedächtnismaterial enthalten und bei Temperaturänderung eine vordefinierte Form annehmen, ist dem Fachmann bekannt und beispielsweise in WO 01/45571 beschrieben.

NITINOL weist für die Zwecke der Erfindung vorteilhafte Feder-und Korrosionseigenschaften auf und ist überdies als medizinischer Werkstoff zugelassen. NITINOL ist in verschiedenen Ausführungen Zusammensetzungen erhältlich, die sich beispielsweise hinsichtlich ihrer Dichten oder Flexibilitäten unterscheiden. Der Fachmann kann die geeignete Zusammensetzung anhand der im Rahmen der Erfindung erforderlichen Dimensionen und Federeigenschaften durch Routineversuche auswählen.

In bevorzugten Ausführungsformen ist in der Einsetzform der distale Abschnitt in einem Winkel zwischen 15° und 75°, bevorzugt zwischen 20° und 60°, besonders bevorzugt zwischen 30° und 50° relativ zum proximalen Abschnitt angeordnet. Diese Winkelbereiche entsprechen den üblichen durch die menschliche Anatomie vorgegebenen Winkeln zwischen dem harten Gaumen und dem weichen Gaumen.

Das Implantat ist zumindest teilweise derart elastisch ausgebildet, dass der distale Abschnitt relativ zum proximalen Abschnitt federnd ist. Insbesondere kann das Implantat im mittleren Abschnitt derart elastisch ausgebildet sein, dass der distale Abschnitt relativ zum proximalen Abschnitt federnd ist. Dies ermöglicht es, dass sich der weiche Gaumen bei bestimmungsgemäss eingesetztem Implantat in einem gewissen Masse relativ zum harten Gaumen bewegen kann. Dies verhindert einerseits ein Zurückfallen des weichen Gaumens in einer liegenden Position des Patienten, andererseits schränkt es aber auch beispielsweise das Sprechen und Schlucken nicht übermässig ein. Bevorzugt ist das Implantat derart elastisch ausgebildet, dass die beim Schlucken hinter dem weichen Gaumen typischerweise auftretende Drücke in Höhe von etwa 50-100 cm Wassersäule noch durch eine durchschnittlich ausgebildete Gaumenmuskulatur überwunden werden können. Das erfindungsgemässe Implantat führt nicht zu einer blossen Versteifung, sondern verleiht dem weichen Gaumen eine erhöhte Spannkraft, und führt den weichen Gaumen in eine natürliche Ruheposition, ähnlich wie dies bei Menschen ohne OSAS durch den weichen Gaumen selbst erreicht wird.

Gemäss einer bevorzugten Ausführungsform ist das Implantat im Wesentlichen streifenförmig ausgebildet. Ein streifenförmiges Implantat lässt sich besonders einfach in einen Gaumen implantieren.

Gemäss bevorzugten Ausführungsformen sind ein proximale Ende des proximalen Abschnitts und/oder ein distales Ende des distalen Abschnitts abgerundet. Auf diese Weise können scharfe Kanten vermieden werden, welche beim Einsetzen des Implantats zu unbeabsichtigten Verletzungen des Gaumens führen könnten oder sich später störend für den Patienten auswirken könnten. Insbesondere können das proximale Ende und/oder das distale Ende innerhalb einer Ebene abgerundet sein, welche senkrecht zu einer Schnittebene verläuft, die sich in der Einsetzform durch den proximalen Abschnitt und den distalen Abschnitt erstreckt.

Bevorzugt weist der proximale Abschnitt eine Länge von 15 mm bis 30 mm, bevorzugt von 20 mm bis 25 mm auf. Unter der Länge des proximalen Abschnitts wird dabei dessen Ausdehnung in Richtung auf den mittleren Abschnitt und in einer mittigen Schnittebene verstanden, welche sich durch den proximalen Abschnitt und den distalen Abschnitt erstreckt. Eine derartige Länge des proximalen Abschnitts ermöglicht einerseits eine ausreichende Festlegung am harten Gaumen, insbesondere wenn der proximale Abschnitt entlang seiner gesamten Länge am harten Gaumen festgelegt wird. Andererseits ist die Länge des proximalen Abschnitts damit derart gewählt, dass sie von einem Patienten nicht als übermässig störend angesehen wird.

Weiterhin bevorzugt weist der distale Abschnitt eine Länge 15 mm bis 25 mm, bevorzugt von 18 mm bis 22 mm, besonders bevorzugt von 19 bis 21 mm auf. Auch die Länge des distalen Abschnitts wird als dessen Ausdehnung in Richtung auf den mittleren Abschnitt und in einer mittigen Schnittebene verstanden, welche sich durch den proximalen Abschnitt und den distalen Abschnitt erstreckt. Ein distaler Abschnitt mit einer derartigen Länge ist an den weichen Gaumen im parauvulären Bereich eines erwachsenen Patienten mit durchschnittlicher Anatomie angepasst.

Bei Implantaten, welche für ein medianes Einsetzen bis an oder in die Uvula vorgesehen sind, weist der distale Abschnitt bevorzugt eine Länge 15 mm bis 35 mm, besonders bevorzugt von 20 mm bis 30 mm auf.

Ebenfalls bevorzugt weist das Implantat eine Breite zwischen 1 mm und 8 mm, bevorzugt zwischen 1,5 mm und 5,5 mm, besonders bevorzugt zwischen 2 mm und 5 mm auf. Die Breite des Implantats wird dabei senkrecht zu einer Schnittebene verstanden, welche sich durch den proximalen Abschnitt und den distalen Abschnitt erstreckt.

Ebenfalls bevorzugt weist das Implantat eine Dicke von 0,02 mm bis 3 mm auf, welche in Abhängigkeit des Materials des Implantats gewählt wird (s. u.). Bei der Dicke handelt es sich dabei um die Ausdehnung des Implantats senkrecht zur oben definierten Längenausdehnung und in einer Schnittebene, welche sich in der Einsetzform durch den proximalen Abschnitt und den distalen Abschnitt erstreckt.

Bei einigen Ausführungsformen kann die Dicke des Implantats zumindest im distalen Abschnitt in Richtung eines distalen Endes des Implantats abnehmen. Diese Form des Implantats ist besonders gut an die Form des weichen Gaumens angepasst. Darüber hinaus kann hierdurch erzielt werden, dass das Implantat in der Nähe seines distalen Endes biegbarer ist als im Bereich des mittleren Abschnitts oder des proximalen Abschnitts.

In einigen Ausführungsformen ist vorgesehen, dass zumindest ein Teil des Implantats eine Einwachstruktur zum Einwachsen von Körpergewebe aufweist. Ein solches Einwachsen von Körpergewebe führt zu einer besseren Verbindung zwischen Implantat und Gaumen. Insbesondere kann die Einwachsstruktur eine Textur und/oder mindestens eine Perforation enthalten. Bevorzugt weist der proximale Abschnitt und/oder der distale Abschnitt eine Einwachsstruktur auf.

Alternativ oder zusätzlich kann das Implantat Mittel zum Verbinden mit dem harten Gaumen aufweisen, insbesondere mindestens eine Aufnahmeöffnung für eine Knochenschraube. Bevorzugt enthält der proximale Abschnitt des Implantats mindestens eine solche Aufnahmeöffnung. Durch eine dort angeordnete Aufnahmeöffnung kann eine Knochenschraube eingesetzt am harten Gaumen fixiert werden. Dies garantiert eine besonders stabile Fixierung des Implantats mit dem weichen Gaumen. Diese Fixierung kann dabei nur vorübergehend erfolgen (beispielsweise nur bis zur gesicherten Wundheilung) oder auch permanent.

Überdies kann der distale Abschnitt mindestens einen Widerhaken umfassen, welcher sich vom distalen Ende des Implantats wegerstreckt. Ein derartiger Widerhaken dient der besseren Verankerung des distalen Abschnitts im weichen Gaumen eines Patienten. Weiterhin kann er beim Einsetzen des Implantats verhindern, dass das Implantat disloziert. Alternativ oder zusätzlich kann auch der proximale Abschnitt einen derartigen Widerhaken enthalten, mit dessen Hilfe eine Dislokation des Implantats verhindert werden kann.

In einigen Ausführungsformen umfasst das Implantat ein insbesondere biokompatibles Metall oder besteht aus einem insbesondere biokompatibles Metall. Insbesondere kann es sich um eine Metalllegierung mit Federeigenschaften handeln, wie beispielsweise NI-VAFLEXO 45/18 (einen aushärtbaren Federwerkstoff auf Kobalt-Nickel-Chrom-Basis, erhältlich von der VACUUMSCHMELZE GmbH und Co. KG, Hanau, Deutschland), NITINOL (eine Formgedächtnis-Legierung auf Nickel-Titan-Basis, erhältlich beispielsweise von Johnson Matthey & Brandenberger AG, Zürich, Schweiz) oder MP35N (eine Nickel-Kobalt-Legierung, erhältlich von der Firma Hempel Special Metals AG, Dübendorf, Schweiz). Sowohl NITINOL als auch MP35N weisen für die Zwecke der Erfindung vorteilhafte Feder-und Korrosionseigenschaften auf und sind überdies als medizinische Werkstoffe zugelassen. Implantate, die Metall umfassen oder daraus bestehen, können beispielsweise durch Lasern, Ätzen oder Stanzen hergestellt werden.

In den Ausführungsformen, in welchen das Implantat ein Metall umfasst oder daraus besteht, hat es bevorzugt eine Dicke von 0,05 mm bis 0,5 mm, besonders bevorzugt von 0,09 mm bis 0,1 mm. Enthält ein derartiges Implantat eine Textur, so kann diese beispielsweise durch Sandstrahlen oder Ätzen gebildet werden, so wie dies an sich beispielsweise bei der Herstellung von Zahnimplantaten bekannt ist. Alternativ können auch eine oder mehrere Perforationen vorliegen, die beispielsweise durch Stanzen eingebracht sein können. Weiterhin kann die Einwachsstruktur auch eine netz- und/oder gitterartige Struktur umfassen. Überdies kann die Oberfläche des Implantats auch variabel aufgeraut sein.

Alternativ oder zusätzlich kann das Implantat einen insbesondere biokompatiblen Kunststoff umfassen oder daraus bestehen. Mögliche biokompatible Kunststoffe sind dabei PBI, PI, TPI, PAI, PEK, PEEK, LCP, PPS, PES, PPSU, PTFE, PFA, PEI, PSU, ETFE, PCTFE, PPP, PC-HT, PVDF, PA 46, PA 6/6T, PC, PET, PA 66, 6-3-T, PBT, PA 6, POM, PMP, PA 12, PA 11, PPE, PMMA, PS, ABS, SAN, PP, PE. Bevorzugt hat der Kunststoff Dauerfestigkeitseigenschaften, d. h. der Kunststoff kann dynamische Belastungen ohne nennenswerte Ermüdungserscheinungen oder Ausfallerscheinungen ertragen und seine Elastizität und Festigkeit dauerhaft behalten.

Erfindungsgemässe Implantate, welche diese Kunststoffe umfassen oder daraus bestehen, können beispielsweise als Spritzgussteil produziert werden.

Gemäss einer möglichen Ausführungsform umfasst das Implantat im Innenbereich einen Kern aus einem Metall und im Aussenbereich eine Umhüllung aus einem Kunststoff. In diesen Ausführungsformen sorgt der Kern aus Metall im Wesentlichen für die gewünschte Biegefestigkeit und Federeigenschaft des Implantats, während die Umhüllung für die Flexibilität der Oberfläche sorgt und damit eine bessere Anpassbarkeit an den Gaumen sowie verbesserte Biokompatibilität garantiert. Eine derartige Umhüllung ist besonders geeignet, falls das Metall zwar für die Erfindung geeignete Federeigenschaften besitzt, aber selbst nicht biokompatibel ist. Der Kern kann hierbei eine Metalllegierung umfassen oder aus einer solchen bestehen, wie beispielsweise eine der oben genannten. Auch die Umhüllung kann aus einen der oben genannten Kunststoffe umfassen oder aus einem solchen bestehen.

In den Ausführungsformen, in welchen das Implantat einen biokompatiblen Kunststoff umfasst oder daraus besteht oder einen Kern aus Metall und eine Umhüllung aus Kunststoff umfasst, hat das Implantat bevorzugt eine Dicke von 0,1 mm bis 3 mm, bevorzugt von 0,2 mm bis 1,5 mm, besonders bevorzugt von 0,3 mm bis 0,6 mm.

In den Ausführungsformen, in welchen das Implantat einen biokompatiblen Kunststoff umfasst oder daraus besteht oder einen Kern aus Metall und eine Umhüllung aus Kunststoff umfasst, können beliebige Strukturen in das für die Herstellung des Implantats verwendete Spritzgusswerkzeug eingearbeitet sein, wodurch eine Einwachsstruktur im Implantat erzielt wird. Alternativ kann der Kunststoff auch wie Metalle nachträglich oberflächlich bearbeitet werden. Alternativ kann die Oberfläche des Implantats auch ein Fasergeflecht aus einem biokompatiblen Kunststoff aufweisen.

Ein weiterer Aspekt der Erfindung betrifft ein Implantatset mit mindestens einem ersten erfindungsgemässen Implantat. In einer ersten Ausführungsform enthält das Implantatset mindestens ein zweites Implantat, welches von dem ersten Implantat verschieden ist. Die Implantate können sich dabei beispielsweise nach ihren Dimensionen (Länge, Breite, Dicke, Krümmungen und/oder Winkel in der Einsetzform), nach ihren Materialien und/oder nach ihren Biegefestigkeiten unterscheiden. Ein derartiges Implantatset erlaubt es dem Chirurgen, je nach Bedarf und individueller Anatomie des zu behandelnden Patienten ein Implantat mit den gewünschten Eigenschaften auszuwählen.

Zudem betrifft die Erfindung auch ein Auswahlverfahren zum Auswählen eines Implantats aus dem erfindungsgemässen Implantatset, welches die folgenden Schritte enthält:
a) Feststellung der Beschaffenheit des harten Gaumens und des weichen Gaumens eines Patienten, insbesondere der Dimensionen und/oder der elastischen Eigenschaften des harten Gaumens und des weichen Gaumens des Patienten;
b) Auswahl eines Implantats in Abhängigkeit von der Feststellung im Schritt a).

Das Implantat kann beispielsweise anhand der folgenden Kriterien ausgewählt werden:
- Die Länge des proximalen Abschnitts des Implantats beträgt zwischen 10 und 50 % der gesamten horizontalen Länge des harten Gaumens in der medianen oder einer dazu parallelen Ebene, in welcher das Implantat eingesetzt werden soll.
- Die Länge des distalen Abschnitts des Implantats beträgt zwischen 30 % und 90 %, bevorzugt zwischen 60 % und 80 % der Länge des weichen Gaumens in der medianen oder einer dazu parallelen Ebene, in welcher das Implantat eingesetzt werden soll.
- Der Winkel, in dem der distale Abschnitt in der Einsetzform relativ zum proximalen Abschnitt angeordnet ist, weicht vom Winkel zwischen dem harten Gaumen und dem weichen Gaumen in einer stehenden Position des Patienten um weniger als 20°, bevorzugt um weniger als 10°, besonders bevorzugt um weniger als 5° ab.

Die Ausmessungen des Gaumens können dabei jeweils direkt am Patienten oder beispielsweise mittels eines Röntgenbildes bestimmt werden. Die Länge des proximalen Abschnitts wird entsprechend der vorgesehenen Positionierung des Implantats bestimmt. Soll das Implantat beispielsweise parauvulär eingesetzt werden, so wird die Länge des weichen Gaumens ebenfalls parauvulär bestimmt, wobei die Uvula nicht zur Länge beiträgt.

Weitere mögliche Kriterien, anhand deren das Implantat ausgewählt werden kann, umfassen
- die Dicke des weichen Gaumens, welche beispielsweise mittels eines Röntgenbildes festgestellt werden kann;
- das Ergebnis einer Palpation des weichen Gaumens;
- die Dicke und Konfiguration des Knochens des harten Gaumens, welche beispielsweise durch ein Röntgenbild festgestellt werden kann;
- die Dicke und Beschaffenheit der Schleimhaut über dem harten Gaumen, welche das Ergebnis von Inspektion und Palpation sein kann oder welche beispielsweise durch ein Röntgenbild festgestellt werden kann;
- den Druckgradienten hinter dem weichen Gaumen, welcher beispielsweise durch eine nächtliche Manometrie der oberen Luftwege festgestellt werden kann;
- die Ausprägung der Obstruktion, welche beispielsweise durch eine medikamentös-induzierte Schlafendoskopie festgestellt werden kann;

Überdies kann die Auswahl eines Implantats auch mit Hilfe eines so genannten "Sizers" getroffen werden. Dabei handelt es sich um ein Dummy-Implantat, welches zur Grössenabschätzung temporär auf den Gaumen gelegt wird. Die Verwendung solcher Dummy-Implantate zur Grössenbestimmung an sich ist beispielsweise bei der Auswahl von Gehörknöchelchenprothesen üblich.

Ein erfindungsgemässes Implantat kann beispielsweise gemäss dem folgenden Einsetzverfahren in einen menschlichen Gaumen eingesetzt werden:
a) Durchführen einer insbesondere transversalen Inzision des weichen Gaumens eines Patienten;
b) Einführung des Implantats durch die Inzision.

Das Implantat kann beispielsweise mit einer Einsetzvorrichtung, beispielsweise mit einem Trokarsystem oder einem ähnlichen System eingeführt werden. Die Länge der Inzision wird dabei auf die Breite des Implantats abgestimmt. Bevorzugt ist daher die Länge der Inzision in etwa gleich gross wie die Breite des Implantats. Die Inzision wird an einer Stelle durchgeführt, deren Abstand vom Übergang des harten Gaumens in den harten Gaumen auf die Länge des distalen Abschnitts des Implantats abgestimmt ist, ihr insbesondere etwa gleich ist. Optional kann das Implantat mit dem harten Gaumen verbunden werden, beispielsweise durch Einsetzen einer Knochenschraube in eine Aufnahmeöffnung des Implantats und Fixieren am harten Gaumen.

Gemäss einigen Einsetzverfahren wird lediglich ein einziges Implantat in den Gaumen eines Patienten eingesetzt. Bevorzugt wird das Implantat dann etwa median eingesetzt. Gemäss anderen Ausführungsformen des Einsetzverfahrens können auch zwei oder mehr Implantate eingesetzt werden, wobei mindestens eines paramedian eingesetzt wird.

In anderen Ausführungsformen des Implantatsets kann dieses ausser einem ersten erfindungsgemässen Implantat weiterhin mindestens eine Einsetzvorrichtung zum Einsetzen des ersten und/oder zweiten Implantats und/oder mindestens einen wie oben beschriebenen Sizer zur Grössenabschätzung für ein einzusetzendes Implantat enthalten.

Im Folgenden wird die Erfindung anhand von mehreren Ausführungsbeispielen und Zeichnungen dargestellt. Dabei zeigen
- Figuren 1a und b: ein erstes erfindungsgemässes Implantat mit einem geraden proximalen Abschnitt und einem geraden distalen Abschnitt;
- Figur 2: ein weiteres erfindungsgemässes Implantat mit einem geraden distalen Abschnitt, einem ge- krümmten proximalen Abschnitt und einem stär- ker gekrümmten mittleren Abschnitt;
- Figur 3: ein weiteres erfindungsgemässes Implantat mit einer Aufnahmeöffnung für eine Knochenschraube und einer Einwachsstruktur;
- Figur 4: ein weiteres erfindungsgemässes Implantat mit einem Kern aus Metall und einer Umhüllung aus Kunststoff;
- Figuren 5a-c: ein weiteres erfindungsgemässes Implantat mit einem Widerhaken;
- Figuren 6a und b: ein weiteres erfindungsgemässes Implantat mit einem Widerhaken und einer Einschnürung;
- Figur 7: ein weiteres erfindungsgemässes Implantat mit einem Widerhaken und einer Einwachsstruktur;
- Figur 8: ein in einen menschlichen Gaumen eingesetztes erfindungsgemässes Implantat.

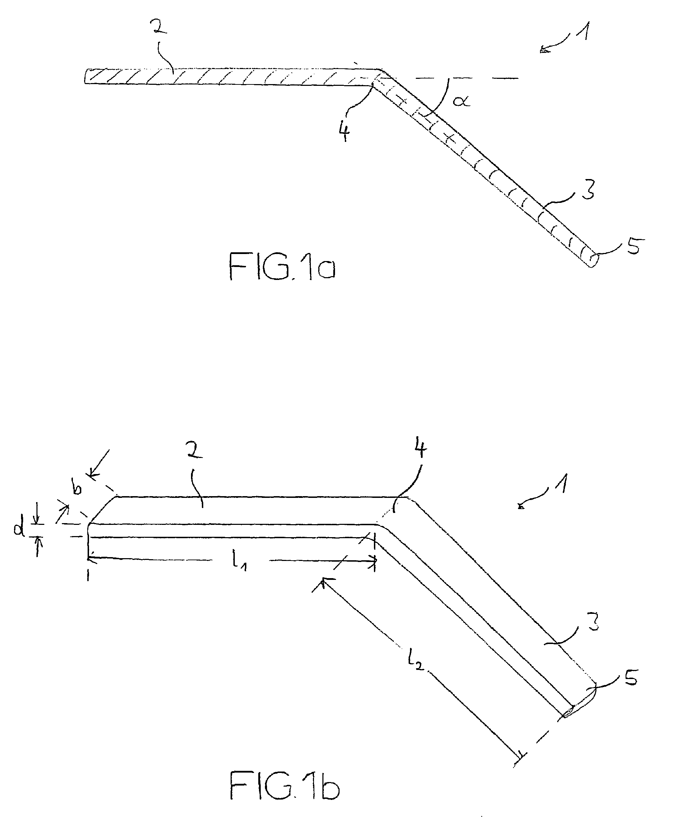

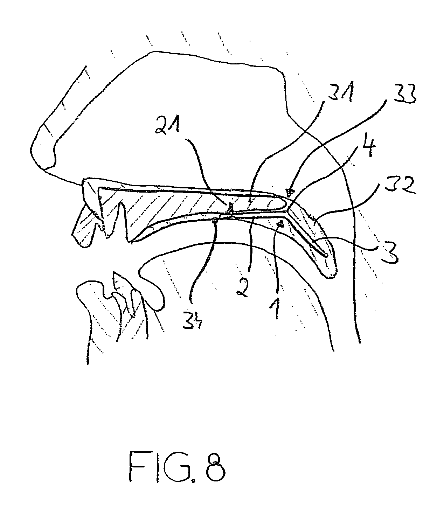

Die Figur 1a zeigt ein erfindungsgemässes Implantat 1 für einen menschlichen Gaumen in einer Schnittansicht. Das Implantat 1 ist streifenförmig und enthält einen proximalen Abschnitt 2, einen distalen Abschnitt 3 und einen mittleren Abschnitt 4, welcher sich zwischen dem proximalen Abschnitt 2 und dem distalen Abschnitt 3 erstreckt. Der proximale Abschnitt 2 ist derart dimensioniert, dass er an einem harten Gaumen festlegbar ist (vgl. Figur 8 unten). Der distale Abschnitt 3 ist derart dimensioniert, dass er vollständig in einem weichen Gaumen aufnehmbar ist. In der in Figur 1a dargestellten Einsetzform ist der distale Abschnitt 3 in einem Winkel α von 40° relativ zum proximalen Abschnitt 2 angeordnet.

Die Zeichenebene der Figur 1a bildet eine Schnittebene des Implantats 1, durch die sich der proximale Abschnitt und der distale Abschnitt 3 erstrecken. In dieser Schnittebene sind sowohl der proximale Abschnitt 2 als auch der distale Abschnitt 3 gerade. Im Sinne der Erfindung wird ihnen also in jedem ihrer Punkte die Krümmung null zugeordnet. Der mittlere Abschnitt 4 wird von einem Knick gebildet, dem eine Krümmung unendlich zugeordnet wird. Im Sinne der Erfindung ist also die Krümmung des mittleren Abschnitts 4 grösser als die Krümmung des proximalen Abschnitts 2 in jedem der Punkte des proximalen Abschnitts 2 und ebenfalls grösser als die Krümmung des distalen Abschnitts 3 in jedem der Punkte des distalen Abschnitts 3.

Die Figur 1b zeigt das Implantat 1 der Figur 1a in einer perspektivischen Ansicht. Sowohl der proximale Abschnitt 2 als auch der distale Abschnitt 3 haben eine Länge l₁ bzw. l₂ von jeweils 20 mm. Die Breite b des Implantats 1 beträgt 2 mm, und seine Dicke d beträgt 1 mm.

Das Implantat 1 gemäss den Figuren 1a und 1b besteht aus einer NITINOL-Metalllegierung. Das Implantat 1 ist derart elastisch ausgebildet, dass der distale Abschnitt 3 relativ zum proximalen Abschnitt 2 federnd ist. Das in einen Gaumen eingesetzte Implantat 1 (vgl. Figur 8) kann somit bewirken, dass der weiche Gaumen eines Patienten als Ganzes versteift wird und relativ zum harten Gaumen im Wesentlichen winkelstabil fixiert wird, wobei jedoch weiterhin eine elastische Bewegung des weichen Gaumens ermöglicht wird. Das Implantat 1 ist insbesondere derart elastisch ausgebildet, dass die beim Schlucken hinter dem weichen Gaumen typischerweise auftretende Drücke in Höhe von etwa 50-100 cm Wassersäule noch durch eine durchschnittlich ausgebildete Gaumenmuskulatur überwunden werden können.

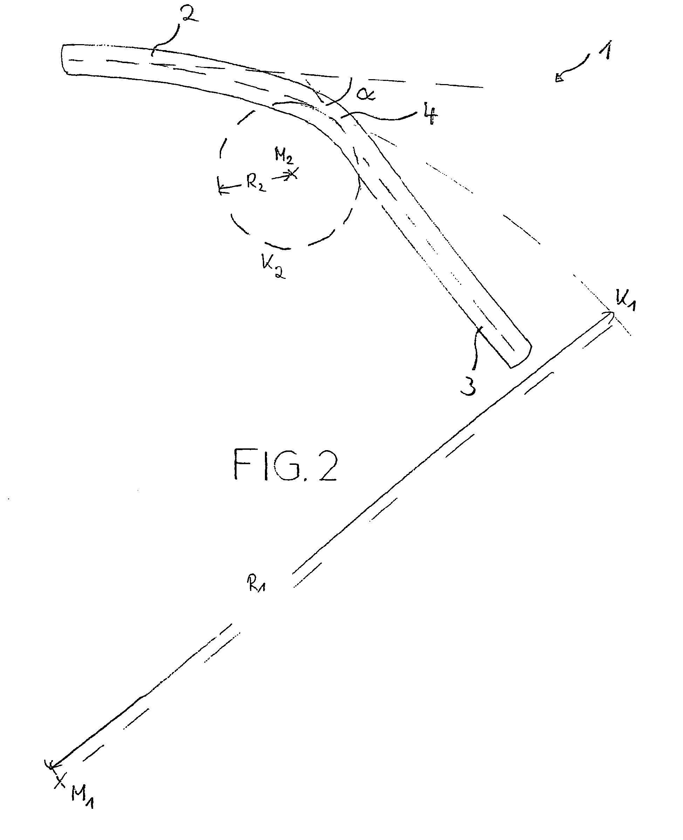

Das in Figur 2 dargestellte Implantat 1 liegt ebenfalls in einer Einsetzform vor und enthält einen geraden distalen Abschnitt 3, jedoch einen gekrümmten proximalen Abschnitt 2. Der proximale Abschnitt 2 weist eine Krümmung von 0,2 cm⁻¹ auf, welche der Inversen des Radius R₁ von 50 mm eines Schmiegekreises K₁ entspricht, welcher sich an den proximalen Abschnitt 2 anschmiegt. Zwischen dem proximalen Abschnitt 2 und dem distalen Abschnitt 3 erstreckt sich ein mittlerer Abschnitt 4, welcher ebenfalls gekrümmt ist. An diesen mittleren Abschnitt 4 des Implantats 1 schmiegt sich ein weiterer Schmiegekreis K₂ mit einem Radius R₂ von 5 mm an, dessen Inverses die Krümmung von 2 cm⁻¹ des Implantats 1 im mittleren Abschnitt 4 bestimmt. Der Radius R₂ ist kleiner als der Radius R₁, sodass die Krümmung in jedem der Punkte des mittleren Abschnitts 4 grösser ist als die Krümmung des proximalen Abschnitts 2 in jedem seiner Punkte und grösser als die Krümmung des distalen Abschnitts 3 in jedem seiner Punkte.

Selbstverständlich ist es auch denkbar und liegt im Rahmen der Erfindung, dass (abweichend von Figur 2) der proximale Abschnitt 2 gerade und der distale Abschnitt 3 gekrümmt ist.

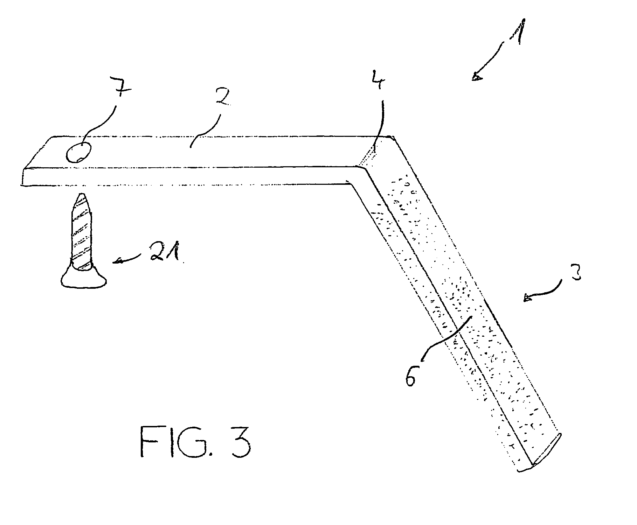

Die Figur 3 zeigt ein weiteres erfindungsgemässes Implantat 1 in einer Einsetzform. Das Implantat 1 enthält einen proximalen Abschnitt 2, einen distalen Abschnitt 3 und einen sich dazwischen befindlichen Knick, welcher einen mittleren Abschnitt 4 bildet. Der proximale Abschnitt weist eine Aufnahmeöffnung für eine Knochenschraube 21 auf, mit deren Hilfe das Implantat 1 an einem harten Gaumen fixiert werden kann. Der distale Abschnitt 3 enthält eine Einwachsstruktur 6, welche von einer Textur gebildet ist. Beim eingesetzten Implantat 1 erlaubt diese Textur das Einwachsen von Körpergewebe, wodurch das Implantat 1 stabiler mit dem Gaumen verbunden wird.

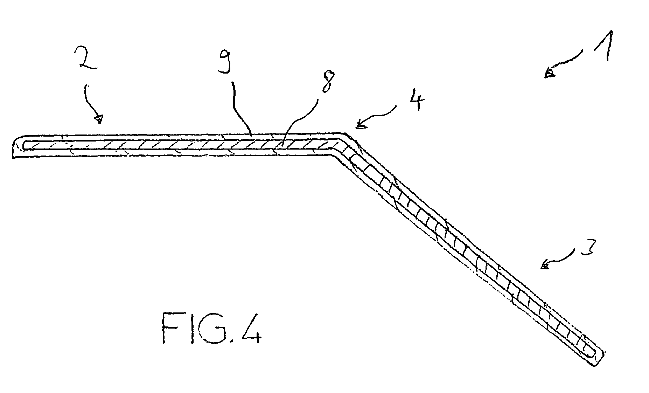

Eine weitere Ausführungsform des erfindungsgemässen Implantats 1 ist in der Schnittansicht der Figur 4 dargestellt. Dieses Implantat 1, welches ebenfalls in einer Einsetzform dargestellt ist, enthält im Innenbereich einen Kern 8 aus NITINOL. Im Aussenbereich ist dieser Kern 8 von einer Umhüllung 9 umhüllt, die aus PPSU besteht.

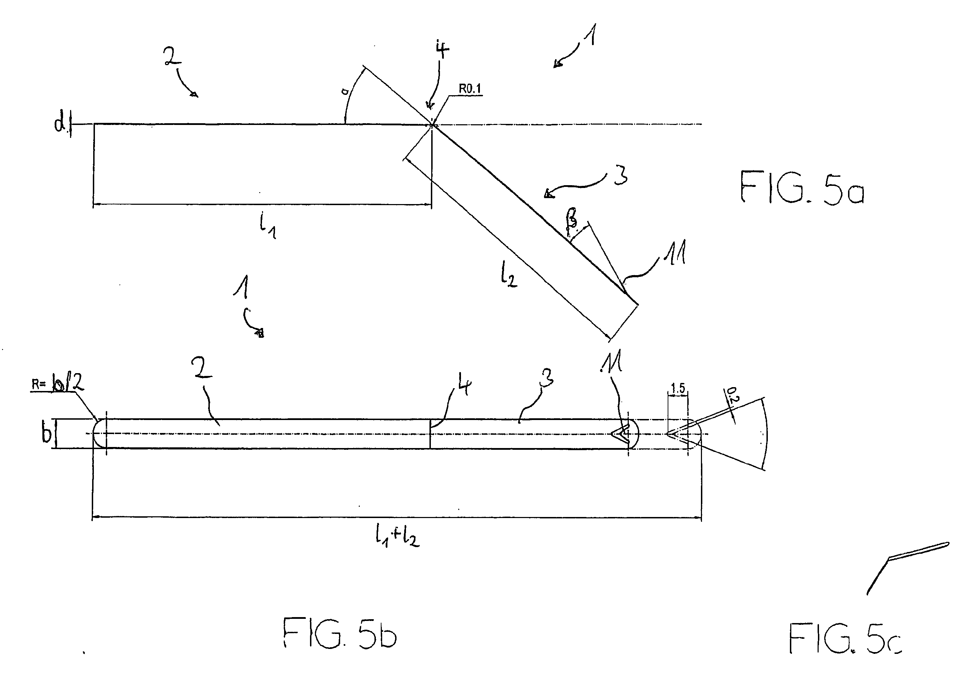

In den Figuren 5a-c ist eine weitere erfindungsgemässe Ausführungsform eines Implantats 1 gezeigt. Figur 5a stellt eine Seitenansicht des Implantats 1 in einer Einsetzform dar. Das Implantat 1 hat eine Dicke d von 0,05 mm. Der proximale Abschnitt 2 hat eine Länge l₁ von 25 mm, während der distale Abschnitt 3 eine Länge l₂ von 20 mm hat. Der zwischen dem proximalen Abschnitt 2 und dem distalen Abschnitt gebildete Winkel α beträgt 40°. Der mittlere Abschnitt 4 ist als Knick mit einem Radius von 0,1 mm ausgebildet, was einem Krümmungsradius von 100 cm⁻¹ entspricht.

In Figur 5a ist ein Widerhaken 11 erkennbar. Dieser erstreckt sich von einem distalen Ende 5 des Implantats 1 unter einem Winkel β von 20° zum distalen Abschnitt 3 weg. Der Widerhaken 11 besitzt die Form eines Dreiecks, dessen eine Seite einen Knick in Richtung der Breite des Implantats 1 bildet und dessen beide andere Seiten freiliegen. Die beiden freiliegenden Seiten laufen an einer Spitze des Widerhakens 11 zusammen. Die beiden freiliegenden Seiten des Widerhakens 11 können durch Einstanzen oder Ätzen zweier Schlitze 13 von 0,2 mm Breite in den flächigen distalen Abschnitt 3 erzeugt werden, die unter einem Winkel y von 40° zueinander angeordnet sind und an einem Punkt zusammenlaufen (vgl. auch Figur 5b). Der Widerhaken 11 dient der verbesserten Verankerung des distalen Abschnitts 3 im weichen Gaumen eines Patienten. Ausserdem kann der Widerhaken 11 eine Dislokation des Implantats 1 beim Einsetzen verhindern. Weiter ist es möglich, einen ähnlichen Widerhaken am proximalen Ende vorzusehen, um eine Dislokation des Implantats zu verhindern.

In Figur 5b ist das Implantat 1 in einer Draufsicht gezeigt. Sowohl das proximale Ende 12 als auch das distale Ende 5 des Implantats 1 sind halbkreisförmig abgerundet. Hierdurch werden scharfe Kanten vermieden, was das Einsetzen des Implantats 1 in einen Gaumen erleichtert und auch für den Patienten angenehmer ist. Die durchgezogene Begrenzungslinie des Implantats 1 in Figur 5b zeigt den Aussenumriss in der geknickten Einsetzform, während die strichpunktierte Begrenzungslinie den Aussenumriss in einer flachen Bereitstellungsform wiedergibt.

Die Figur 5c zeigt das Implantat 1 in einer perspektivischen Ansicht.

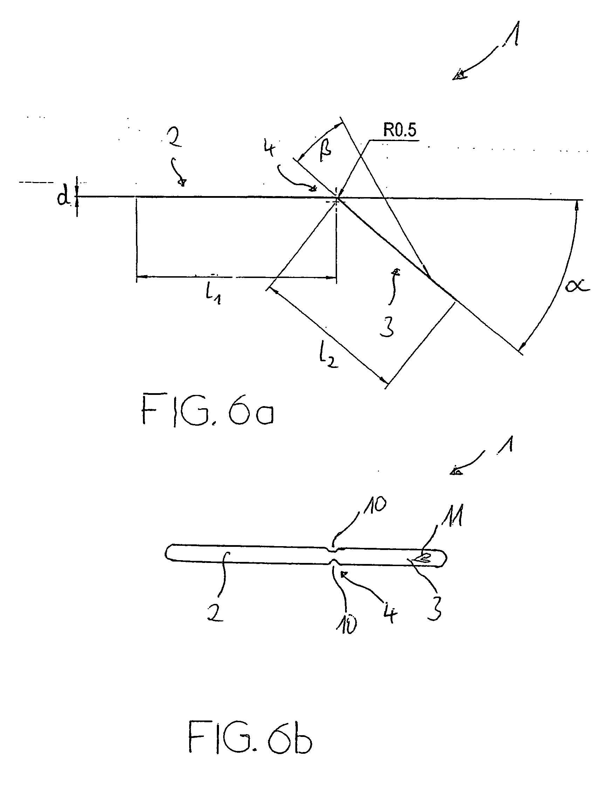

In den Figuren 6a und b ist ein weiteres erfindungsgemässes Implantat 1 in einer Seitenansicht bzw. einer Draufsicht gezeigt. Dieses Implantat 1 unterscheidet sich von dem in den Figuren 5a-c gezeigten durch eine Einschnürung 10 im mittleren Abschnitt 4, welche einen Solldeformationsbereich bildet. Diese Einschnürung 10 definiert eine Position, an welcher das Implantat 1 gebogen und/oder geknickt werden kann, um es von einer Bereitstellungsform in eine Einsetzform zu überführen.

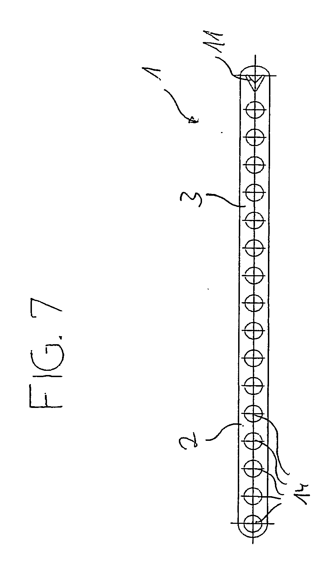

In Figur 7 ist ein weiteres erfindungsgemässes Implantat 1 dargestellt, welches in einer flachen Bereitstellungsposition vorliegt. Aus dieser Bereitstellungsposition kann es durch Biegen oder Knicken in eine Einsetzform gebracht werden. Auch dieses Implantat verfügt über einen Widerhaken 11, welcher mit den in den Figuren 5a-c sowie 6a und b gezeigten übereinstimmt. Ferner enthält das Implantat 1 eine Anzahl von 16 kreisförmigen, äquidistanten Perforationen 14, welche das Implantat durchdringen und eine Einwachsstruktur zum Einwachsen von Körpergewebe bilden, wodurch das Implantat 1 stabiler mit dem Gaumen verbunden werden kann.

Die Figur 8 zeigt in einer Schnittdarstellung einen Teil eines menschlichen Kopfes. Der Gaumen enthält einen harten Gaumen 31 und einen weichen Gaumen 32. In den Gaumen ist ein erfindungsgemässes Implantat 1 in der Einsetzform eingesetzt. Dabei ist der proximale Abschnitt 2 des Implantats 1 entlang seiner gesamten Länge am harten Gaumen 31 festgelegt. Dies ist möglich aufgrund der geringen Krümmung des proximalen Abschnitts 2 im Vergleich zu der des mittleren Abschnitts 4. Der proximale Abschnitt 2 ist zusätzlich mit Hilfe einer Knochenschraube 21 am harten Gaumen 31 fixiert. Der distale Abschnitt 3 des Implantats 1 ist vollständig im weichen Gaumen 32 aufgenommen und erstreckt sich in etwa entlang zwei Dritteln von dessen Länge. Zwischen dem proximalen Abschnitt 2 und dem distalen Abschnitt 3 weist das Implantat 1 eine Kante auf, welche einen mittleren Abschnitt 4 bildet. Dieser mittlere Abschnitt 4 ist im Bereich des Übergangs 33 vom harten Gaumen 31 in den weichen Gaumen 32 angeordnet. Zum Einsetzen des Implantats 1 kann dieses in eine zuvor durchgeführte Inzision 34 eingeführt werden.

Aufgrund der Festlegung des proximalen Abschnitts 2 am harten Gaumen 31 ist auch der distale Abschnitt 3 des Implantats 1 und somit der weiche Gaumen 32 unter einem Winkel relativ zum harten Gaumen 31 gehalten. In einer liegenden Position des Patienten kann somit der weiche Gaumen 32 nicht in beeinträchtigender Weise in den Rachenraum zurückfallen. Aufgrund der Elastizität des Implantats 1 ist jedoch der distale Abschnitt 3 relativ zum proximalen Abschnitt 2 federnd gelagert, sodass auch der weiche Gaumen 32 relativ zum harten Gaumen 31 federnd gelagert ist. So werden beispielsweise das Sprechen und Schlucken nicht wesentlich behindert.

Ein erfindungsgemässes Implantatset, welches nicht zeichnerisch darstellt ist, kann beispielsweise Implantate mit einigen oder allen möglichen Kombinationen der folgenden Dimensionen enthalten:
- Breite des Implantats: 2 mm, 3 mm, 5 mm;
- Länge des proximalen Abschnitts: 20 mm, 25 mm;
- Länge des distalen Abschnitts: 20 mm;
- Winkel zwischen proximalem und distalem Abschnitt: 30°, 35°, 40°, 45°, 50;

Insgesamt ergeben sich somit 3 mal 2 mal 5, also 30 mögliche Varianten des Implantats innerhalb dieses Implantatsets.

## Patentansprüche

1. Implantat (1) für einen insbesondere menschlichen Gaumen, enthaltend
- einen distalen Abschnitt (3), welcher derart dimensioniert ist, dass er vollständig in einem weichen Gaumen (32) aufnehmbar ist,
- einen proximalen Abschnitt (2), welcher derart dimensioniert ist, dass er an einem harten Gaumen (31) festlegbar ist,
- einen mittleren Abschnitt (4), welcher sich zwischen dem proximalen Abschnitt (2) und dem distalen Abschnitt (3) erstreckt,
**dadurch gekennzeichnet, dass**
das Implantat zumindest teilweise, insbesondere im mittleren Abschnitt (4) derart elastisch ausgebildet ist, dass der distale Abschnitt (3) relativ zum proximalen Abschnitt (2) federnd ist.

2. Implantat (1) gemäss Anspruch 1,
**dadurch gekennzeichnet, dass**
das Implantat (1) in einer Einsetzform vorliegt oder in eine Einsetzform bringbar ist, wobei in der Einsetzform
- der distale Abschnitt (3) in einem Dinkel (α) relativ zum proximalen Abschnitt (2) angeordnet ist und
- in einer sich durch den proximalen Abschnitt (2) und den distalen Abschnitt (3) erstreckenden Schnittebene die Krümmung des mittleren Abschnitts (4) in jedem der Punkte des mittleren Abschnitts (4) grösser ist als
- die Krümmung des proximalen Abschnitts (2) in jedem der Punkte des proximalen Abschnitts (2) und
- die Krümmung des distalen Abschnitts (3) in jedem der Punkte des distalen Abschnitts (3).

3. Implantat (1) gemäss einem der Ansprüche 1 und 2,
**dadurch gekennzeichnet, dass**
der mittlere Abschnitt (4) einen Solldeformationsbereich, insbesondere mindestens eine Einschnürung (10) aufweist, an dem der mittlere Abschnitt (4) insbesondere plastisch deformierbar, insbesondere biegbar und/oder knickbar ist.

4. Implantat (1) gemäss einem der Ansprüche 1 bis 3,
**dadurch gekennzeichnet, dass**
es ein Formgedächtnismaterial, insbesondere NITINOL, umfasst oder daraus besteht und bei einer Einsetztemperatur zwischen 35 °C und 40 °C, insbesondere zwischen 36 °C und 37 °C, in der Einsetzform vorliegt.

5. Implantat (1) gemäss einem der Ansprüche 2 bis 4,
**dadurch gekennzeichnet, dass**
der proximale Abschnitt (2) in der Einsetzform in einem Winkel (α) zwischen 15° und 75°, bevorzugt zwischen 2C° und 60°, besonders bevorzugt zwischen 30° und 50° relativ zum distalen Abschnitt (3) angeordnet ist.

6. Implantat (1) gemäss einem der Ansprüche 1 bis 5,
**dadurch gekennzeichnet, dass**
es im Wesentlichen streifenförmig ausgebildet ist.

7. Implantat (1) gemäss einem der Ansprüche 1 bis 6,
**dadurch gekennzeichnet, dass**
der proximale Abschnitt (2) eine Länge (l₁) von 15 mm bis 30 mm, bevorzugt von 20 mm bis 25 mm aufweist.

8. Implantat (1) gemäss einem der Ansprüche 1 bis 7,
**dadurch gekennzeichnet, dass**
der distale Abschnitt (3) eine Länge (l₂) von 15 mm bis 25 mm, bevorzugt von 18 mm bis 22 mm, besonders bevorzugt von 19 mm bis 21 mm aufweist.

9. Implantat (1) gemäss einem der Ansprüche 1 bis 8,
**dadurch gekennzeichnet, dass**
die Dicke (d) des Implantats (1) zumindest im distalen Abschnitt (3) in Richtung eines distalen Endes (5) des Implantats (1) abnimmt.

10. Implantat (1) gemäss einem der Ansprüche 1 bis 9,
**dadurch gekennzeichnet, dass**
zumindest ein Teil des Implantats (1) eine Einwachsstruktur (6) zum Einwachsen von Körpergewebe aufweist, insbesondere eine Textur und/oder mindestens eine Perforation.

11. Implantat (1) gemäss einem der Ansprüche 1 bis 10,
**dadurch gekennzeichnet, dass**
das Implantat (1), insbesondere der proximale Abschnitt (2) Mittel zum Verbinden mit dem harten Gaumen (31) aufweist, insbesondere mindestens eine Aufnahmeöffnung (7) für eine Knochenschraube (21).

12. Implantat (1) gemäss einem der Ansprüche 1 bis 11,
**dadurch gekennzeichnet, dass**
es ein insbesondere biokompatibles Metall, insbesondere einne biokompatible Metalllegierung umfasst oder daraus besteht.

13. Implantat (1) gemäss einem der Ansprüche 1 bis 12,
**dadurch gekennzeichnet, dass**
es einen insbesondere biokompatiblen Kunststoff umfasst oder daraus besteht.

14. Implantat (1) gemäss einem der Ansprüche 12 und 13,
**dadurch gekennzeichnet, dass**
es
- im Innenbereich einen Kern (8) aus einem Metall, insbesondere aus einer Metalllegierung, sowie
- im Aussenbereich eine Umhüllung (9) aus einem Kunststoff
umfasst.

15. Implantat (1) gemäss einem der Ansprüche 1 bis 14,
**dadurch gekennzeichnet, dass**
der distale Abschnitt (3) und/oder der proximale Abschnitt (2) mindestens einen Widerhaken (11) umfasst, welcher sich vom distalen Ende (3) des Implantats (1) wegerstreckt.

16. Implantatset, enthaltend mindestens ein erstes Implantat (1) gemäss einem der Ansprüche 1 bis 15 sowie
- mindestens ein zweites Implantat (1) gemäss einem der Ansprüche 1 bis 15, wobei das zweite Implantat (1) von dem ersten Implantat (1) verschieden ist, und/oder
- mindestens eine Einsetzvorrichtung zum Einsetzen des ersten und/oder zweiten Implantats (1) und/oder
- mindestens einen Sizer zur Grössenabschätzung für ein einzusetzendes Implantat (1).

17. Auswahlverfahren zum Auswählen eines Implantats (1) aus einem Implantatset gemäss Anspruch 16, **gekennzeichnet durch** die folgenden Schritte:
a) Feststellung der Beschaffenheit des harten Gaumens (31) und des weichen Gaumens (32) eines Patienten, insbesondere der Dimensionen und/oder der elastischen Eigenschaften des harten Gaumens (31) und des weichen Gaumens (32) des Patienten;
b) Auswahl eines Implantates (1) in Abhängigkeit von der Feststellung in Schritt a).

## Claims

1. Implant (1) for a palate, in particular a human palate, comprising
- a distal portion (3), which is dimensioned in such a way that it can be completely accommodated in a soft palate (32),
- a proximal portion (2), which is dimensioned in such a way that it can be fixed to a hard palate (31), and
- a middle portion (4), which extends between the proximal portion (2) and the distal portion (3),
**characterized in that** the implant is at least partially elastically formed, in particular in the middle portion (4), in such a way that the distal portion (3) is resilient in relation to the proximal portion (2).

2. Implant (1) according to Claim 1, **characterized in that** the implant (1) is in an insertable form or can be brought into an insertable form, wherein, in the insertable form,
- the distal portion (3) is arranged at an angle (α) in relation to the proximal portion (2) and
- in a sectional plane extending through the proximal portion (2) and the distal portion (3), the curvature of the middle portion (4) at each of the points of the middle portion (4) is greater than
- the curvature of the proximal portion (2) at each of the points of the proximal portion (2) and
- the curvature of the distal portion (3) at each of the points of the distal portion (3).

3. Implant (1) according to either of Claims 1 and 2, **characterized in that** the middle portion (4) has an intended deformation region, in particular at least one constriction (10), at which the middle portion (4) can be deformed, in particular plastically deformed, in particular can be bent and/or can be kinked.

4. Implant (1) according to one of Claims 1 to 3, **characterized in that** it comprises a shape memory material, in particular NITINOL, or consists thereof, and at an insertion temperature of between 35°C and 40°C, in particular between 36°C and 37°C, is in the insertable form.

5. Implant (1) according to one of Claims 2 to 4, **characterized in that**, in the insertable form, the proximal portion (2) is arranged at an angle (α) of between 15° and 75°, preferably between 20° and 60°, particularly preferably between 30° and 50°, in relation to the distal portion (3).

6. Implant (1) according to one of Claims 1 to 5, **characterized in that** it is substantially in the form of a strip.

7. Implant (1) according to one of Claims 1 to 6, **characterized in that** the proximal portion (2) has a length (l₁) of 15 mm to 30 mm, preferably of 20 mm to 25 mm.

8. Implant (1) according to one of Claims 1 to 7, **characterized in that** the distal portion (3) has a length (l₂) of 15 mm to 25 mm, preferably of 18 mm to 22 mm, particularly preferably of 19 mm to 21 mm.

9. Implant (1) according to one of Claims 1 to 8, **characterized in that** the thickness (d) of the implant (1) decreases at least in the distal portion (3) in the direction of the distal end (5) of the implant (1).

10. Implant (1) according to one of Claims 1 to 9, **characterized in that** at least part of the implant (1) has an ingrowth structure (6) for the growing in of body tissue, in particular a texture and/or at least one perforation.

11. Implant (1) according to one of Claims 1 to 10, **characterized in that** the implant (1), in particular the proximal portion (2), has means for connecting to the hard palate (31), in particular at least one receiving opening (7) for a bone screw (21).

12. Implant (1) according to one of Claims 1 to 11, **characterized in that** it comprises a metal, in particular a biocompatible metal, in particular a biocompatible metal alloy, or consists thereof.

13. Implant (1) according to one of Claims 1 to 12, **characterized in that** it comprises a plastic, in particular a biocompatible plastic, or consists thereof.

14. Implant (1) according to either of Claims 12 and 13, **characterized in that** it comprises
- a core (8) of a metal, in particular of a metal alloy, in the inner region and
- a shell (9) of a plastic in the outer region.

15. Implant (1) according to one of Claims 1 to 14, **characterized in that** the distal portion (3) and/or the proximal portion (2) comprise(s) at least one barb (11), which extends away from the distal end (5) of the implant (1).

16. Set of implants, including at least a first implant (1) as claimed in one of Claims 1 to 15 as well as
- at least a second implant (1) as claimed in one of Claims 1 to 15, the second implant (1) being different from the first implant (1), and/or
- at least one insertion device for inserting the first and/or second implant (1) and/or
- at least one sizer for estimating the size of an implant (1) to be inserted.

17. Selection process for selecting an implant (1) from a set of implants as claimed in Claim 16, **characterized by** the following steps:
a) establishing the condition of the hard palate (31) and the soft palate (32) of a patient, in particular the dimensions and/or the elastic properties of the hard palate (31) and the soft palate (32) of the patient;
b) selecting an implant (1) in dependence on the finding established in step a).

## Revendications

1. Implant (1) pour un palais en particulier humain, comportant
- une partie distale (3), qui est dimensionnée de telle manière qu'elle puisse être entièrement logée dans un palais mou (32),
- une partie proximale (2), qui est dimensionnée de telle manière qu'elle puisse être fixée sur un palais dur (31),
- une partie médiale (4), qui s'étend entre la partie proximale (2) et la partie distale (3),
**caractérisé en ce que** l'implant est réalisé au moins partiellement sous forme élastique, en particulier dans la partie médiale (4), de telle manière que la partie distale (3) fasse ressort par rapport à la partie proximale (2).

2. Implant (1) selon la revendication 1, **caractérisé en ce que** l'implant (1) se présente sous une forme apte à la pose ou peut être mis sous une forme apte à la pose, dans lequel, dans la forme apte à la pose,
- la partie distale (3) est disposée avec un angle (α) par rapport à la partie proximale (2), et
- dans un plan de coupe s'étendant à travers la partie proximale (2) et la partie distale (3), la courbure de la partie médiale (4) en chacun des points de la partie médiale (4) est plus grande que
- la courbure de la partie proximale (2) en chacun des points de la partie proximale (2), et
- la courbure de la partie distale (3) en chacun des points de la partie distale (3).

3. Implant (1) selon l'une quelconque des revendications 1 et 2, **caractérisé en ce que** la partie médiale (4) présente une zone de déformation préférentielle, en particulier au moins un étranglement (10), dans laquelle la partie médiale (4) est déformable, en particulier flexible et/ou pliable, en particulier de manière plastique.

4. Implant (1) selon l'une quelconque des revendications 1 à 3, **caractérisé en ce qu'**il comprend un matériau à mémoire de forme, en particulier du NITINOL, ou est constitué de celui-ci, et il se présente sous la forme apte à la pose à une température de pose comprise entre 35°C et 40°C, en particulier entre 36°C et 37°C.

5. Implant (1) selon l'une quelconque des revendications 2 à 4, **caractérisé en ce que** la partie proximale (2) est, dans la forme apte à la pose, disposée avec un angle (α) compris entre 15° et 75°, de préférence entre 20° et 60°, et de préférence encore entre 30° et 50° par rapport à la partie distale (3).

6. Implant (1) selon l'une quelconque des revendications 1 à 5, **caractérisé en ce qu'**il est réalisé essentiellement en forme de bande.

7. Implant (1) selon l'une quelconque des revendications 1 à 6, **caractérisé en ce que** la partie proximale (2) présente une longueur (l₁) de 15 mm à 30 mm, de préférence de 20 mm à 25 mm.

8. Implant (1) selon l'une quelconque des revendications 1 à 7, **caractérisé en ce que** la partie distale (3) présente une longueur (l₂) de 15 mm à 25 mm, de préférence de 18 mm à 22 mm, et de préférence encore de 19 mm à 21 mm.

9. Implant (1) selon l'une quelconque des revendications 1 à 8, **caractérisé en ce que** l'épaisseur (d) de l'implant (1) diminue au moins dans la partie distale (3) en direction d'une extrémité distale (5) de l'implant (1).

10. Implant (1) selon l'une quelconque des revendications 1 à 9, **caractérisé en ce qu'**au moins une partie de l'implant (1) présente une structure de croissance (6) pour la croissance de tissu corporel, en particulier une texture et/ou au moins une perforation.

11. Implant (1) selon l'une quelconque des revendications 1 à 10, **caractérisé en ce que** l'implant (1), en particulier la partie proximale (2), présente des moyens pour la liaison au palais dur (31), en particulier au moins une ouverture de réception (7) pour une vis à os (21).

12. Implant (1) selon l'une quelconque des revendications 1 à 11, **caractérisé en ce qu'**il comprend un métal en particulier biocompatible, en particulier un alliage métallique biocompatible, ou il en est constitué.

13. Implant (1) selon l'une quelconque des revendications 1 à 12, **caractérisé en ce qu'**il comprend une matière plastique en particulier biocompatible, ou il en est constitué.

14. Implant (1) selon l'une quelconque des revendications 12 et 13, **caractérisé en ce qu'**il comprend
- dans la région interne un noyau (8) en un métal, en particulier en un alliage métallique, ainsi que
- dans la région externe une gaine (9) en une matière plastique.

15. Implant (1) selon l'une quelconque des revendications 1 à 14, **caractérisé en ce que** la partie distale (3) et/ou la partie proximale (2) comporte au moins un ardillon (11), qui s'étend dans la direction opposée à l'extrémité distale (5) de l'implant (1).

16. Ensemble d'implant, contenant au moins un premier implant (1) selon l'une quelconque des revendications 1 à 15, ainsi que
- au moins un deuxième implant (1) selon l'une quelconque des revendications 1 à 15, dans lequel le deuxième implant (1) est différent du premier implant (1), et/ou
- au moins un dispositif de pose pour la pose du premier et/ou du deuxième implant (1), et/ou
- au moins un gabarit destiné à l'évaluation de la grandeur pour un implant à poser (1).

17. Procédé de sélection pour la sélection d'un implant (1) dans un ensemble d'implant selon la revendication 16, **caractérisé par** les étapes suivantes:
a) détermination de la nature du palais dur (31) et du palais mou (32) d'un patient, en particulier des dimensions et/ou des propriétés élastiques du palais dur (31) et du palais mou (32) du patient;
b) sélection d'un implant (1) en fonction de la détermination effectuée à l'étape a).
